# EUROPEAN PATENT SPECIFICATION

(11) **EP 0 414 304 B1**
(45) Date of publication and mention of the grant of the patent: **02.11.1994**
(21) Application number: 90202169.0
(22) Date of filing: 10.08.1990
(51) Int. Cl.: A01N 63/00, A61K 7/16, A61K 7/48, A61K 7/06

(54) **Aqueous antibacterial compositions comprising surfactant and bacteriophage**
Wässrige Zusammensetzung auf der Basis eines Tensids und eines Bacteriophagen
Composition aqueuse comprenant un agent tesioactif et un bacteriophage

(30) Priority: 21.08.1989 GB 8918983
(43) Date of publication of application: 27.02.1991
(73) Proprietor: UNILEVER N.V., 3013 AL Rotterdam (NL); UNILEVER PLC, London EC4P 4BQ (GB)
(72) Inventor: Jones, Carol Lesley, Bebington, Wirral, Merseyside L63 3JW (GB); Rennie, George Kerr, Bebington, Wirral, Merseyside L63 3JW (GB); Moore, Charles Herbert, Bebington, Wirral, Merseyside L63 3JW (GB)
(74) Representative: Bryant, Tracey

(56) References cited:
- EP-A- 0 102 151
- EP-A- 0 154 549
- DE-A- 2 511 258
- US-A- 4 891 210
- JAPANESE PATENTS GAZETTE Section Ch, Week 8806, 23 March
- 1988 Derwent Publications Ltd., London, GB; Class D, AN 88-039734/06& JP-A-62 298 498 (AGENCY OF IND. SCI.TECH. (KANT MITO)) 15 December 1987
- CHEMICAL PATENTS INDEX, BASIC ABSTRACTS JOURNAL Section Ch, Week 8728, 9September 1987 Derwent Publications Ltd., London, GB; Class C, AN 87-194632/28& JP-A-62 123 104 (JAPANTOBACCO & SALT PUB.) 4 June 1987

## Description

The present invention relates to the use of viruses against aqueous antibacterial compositions comprising surfactant and bacteriophages.

Bacteria occur in a large number of varieties, some of which are pathogenic and may cause disease in animals and humans. In the presence of sufficient amounts of nutrients and under the right conditions, bacteria may grow and multiply at a rapid rate. This is not only undesirable from a hygienic point of view, as it may lead to infections, but also because of accompanying phenomena such as the formation of odour, inorganic scales or other unwelcome deposits.

There are various places where undesirable growth of bacteria may occur. For example, bacteria may multiply on teeth to form plaque in the human or animal mouth which may cause tooth decay or other disease. Another example is the toilet bowl where faecal bacteria, such as E. coli may multiply so creating off-odours and a potential hazard to health. Yet another example is the human skin where infection by P. acnes causes unsightly skin lesions, usually referred to as acne.

Various methods are available to kill micro-organisms or to control their growth. For instance, it is known to disinfect toilet bowls by means of liquid products containing one or more sanitizing agents, such as hypochlorite. Such products often contain surfactants to effect wetting and cleaning. Another example is skin-cleansing products such as soaps which may contain bactericidal compounds. A disadvantage of such systems, particularly those for cleaning the skin, resides in the fact that they may provide an "overkill" by destroying unnecessarily all microbiological life. Other examples are liquid w.c. sanitizing products, whereby most of the chemical used for sanitizing is directly transported from the bowl to the sewage system upon subsequent flushing. Therefore, larger amounts of sanitizer are used that strictly necessary for sanitizing the toilet bowl. This is not only uneconomical, but environmental problems may also result.

It is also known to combat pathogenic bacteria by means of specific chemical substances in the form of pharmaceutical compositions. For some bacteria, however, no suitable chemical compounds are known. Furthermore, most bacteria tend to develop some form of resistance against compounds to which they are continuously exposed, such that higher and higher dosages are required to obtain any bactericidal effect.

In the area of dental care it is known to remove plaque by means of toothpastes comprising small amounts of bactericidal agents such as chlorohexidine. Such toothpastes have the disadvantage that they provide a broad spectrum of anti-microbial activity, and by generally reducing the numbers of oral bacteria so-called opportunistic infections of resistant bacteria or yeasts may be actually promoted.

An alternative to the use of germicidal agents is proposed in UK patent 829266. That document teaches use of bacteriophages to prevent and/or cure infectious diseases which are caused by bacteria, such as dysentery, typhus etcetera. This form of treatment is known as 'phagotherapy' and involves the application of a therapeutic bacteriophage preparation, which is essentially in a solid, dry form. A similar technique is known for the control of insect pests from GB 1441094. JP-A-261354 relates to 'phagotherapy' as applied to eggplants so as to eradicate pathogenic P. solanacearum.

JP-A-62298498 discloses the use of bacteriophages to kill bacteria in seawater-using equipment, particularly water-cooled power plants. It is known that oceanic bacteria cause scale to form on surfaces contacted by seawater and this document discloses that compositions can be prepared which comprise a plurality of 'sponge balls' carrying a suitable bacteriophage.

It is an object of the present invention to provide a bactericidal composition to prevent or control the growth of hygienically undesirable bacteria, by means of which the above-mentioned problems may be avoided.

We have now surprisingly found that it is possible to use bacteriophages to prevent or control the growth of hygienically undesirable bacteria.

Bacteriophages are viruses which are dependent on bacteria as host cells for their propagation. To that end they infect a bacterial cell and induce it to produce large numbers of copies of the infecting phage. When the production of the phages is complete, the bacterial cell bursts open (lysis) and the new phages are set free. The bacterium is thereby destroyed.

Therefore, according to a first aspect of the present invention, there is provided an aqueous antibacterial composition comprising, an nonionic surfactant, a neutral salt and at least 100 particles/ml of a bacteriophage capable of lysing one or more type of said undesirable bacteria.

It was surprising that despite the presence of a surface-active compound, the bacteriophages have an excellent storage stability in the above compositions, i.e. they retain their capacity to infect their host micro-organism for as long as two months at room temperature.

Many different types of bacteriophages have been isolated and described in the literature. They range from relatively simple phages, for example fd, which contains one single stranded DNA molecule, to vary complex nucleic acid-protein assemblies like the T-even phages. Some phages maybe have a narrow host range, i.e. they may be capable of lysing a specific type of bacterium only. Other phages may be capable of infecting and lysing various types of bacteria. Both types of bacteriophages may be used in the present invention, depending on the specific application.

The composition of the present invention requires a compatible medium in which the bacteriophage can be stored for some time without losing its infectivity.

The pH of the medium will generally be from 5 to 9, though compositions having a higher or lower pH are possible, and is preferably buffered. The ionic strength of the medium should be sufficiently high to prevent dissociation of the bacteriophage into its constituents. Dissociation and or denaturation may also occur at high ionic strength and so extremes must be avoided.

Preferably the composition comprises 0.001 to 15.0%, more preferably 0.01 to 5.0% by weight and most preferably 0.1 to 3.0% by weight of a nonionic surfactant.

An important function of the surfactant is that it helps to wet the surface, so that the compositions is properly distributed over the entire surface. Another function is of course that the surfactant helps to solubilise and remove dirt so that bacteria become accessible. Suitable nonionic surfactants are for instance Tween 80, 20, 81 and Dobanols such as Dobanol 23-6.5.

The composition further comprises 0.01 to 2.0% by weight of a neutral salt, and preferably more than 10₃ Particles/ml of a bacteriophage capable of infecting household bacteria, such as E. coli. For the purpose of this invention, household bacteria are defined as the bacteria which occur in the household, in particular faecal bacteria.

As the neutral salt one may advantageously use 0.1 to 1.0% by weight of sodium chloride.

It is especially preferred if the composition also comprises a small amount of a thickening agent such as 250 Natrosol HHBR, a modified cellulose polymer ex Hercules.

The bactericidal composition may be directed against pathogenic skin bacteria such as Propronibacterium acnes or odour producing skin bacteria. To that end it comprises a bacteriophage which is capable of lysing such bacteria. The composition may also be directed against P. ovale and/or other scalp bacteria, in which case it comprises a bacteriophage capable of lysing such bacteria.

Other embodiments of the invention provide a bactericidal composition in the form of a toothpaste or mouth wash composition. This composition comprises an abrasive substance such as silica, a binder and preferably more than 10₂, preferably much more that 10₃ particles per g of a bacteriophage capable of lysing one or more of the types of bacteria involved in caries or gum disease. Examples of such bacteria are S. mutans, bacteroides gingivalis and Haemophilus actinomycetemcomitans.

The compositions according to the invention may include phage-compatible perfumes, flavours, solvents, dyes, pigments, preservatives, bactericides, absorbents, fillers, and other additives which are commonly used for a specific application.

Methods of isolating bacteriophages are known in the art, see for instance Billing, E., (1969), Methods in Microbiology, Vol. 3b, Ed. J.R. Norris and D.W. Ribbons, Acad. Press, p. 315-329. Phages can usually be isolated from cultures of the bacteria to which they are specific. For instance, bacteriophages against enteric bacteria can be isolated from sewage using the enrichment method described by M.H. Adams in Bacteriophages, Interscience, New York (1959).

Phages against oral bacteria are isolated from plaque and saliva, enrichment techniques were found to be less suitable here. Phages against P. acnes can be isolated from infected skin lesions.

Although crude extracts may be used for some applications, it is often desirable to apply the phages in purified form. To that end the phages are separated from the bacterial cell debris, in particular from catabolic enzymes. Separation may be effected by known methods such as filtration, centrifugation or chromatography.

The invention will now be illustrated by means of the following examples.

### EXAMPLES 1-7: Faecal Bacteriophages

### 1. Isolation

Fresh sewage was filtered through a 0.2 um filter to remove bacteria. 0.5 ml of the filtered sewage was spread onto a plate previously seeded with one of the following faecal organisms identified as E. coli, Proteus mirabilis, Vibrio flurvalis, Citrobacter freundii, Enterobacter agglomerans, E. vulneris or Pseudomonas fluorescens. The plate was then dried and incubated overnight at 28°C. After incubation several individual plaques could be seen on the bacterial overlay. These were removed from the agar and inoculated into broth cultures of the sewage organism to produce large quantities of phage specific for this micro-organism, as described below.

Sometimes it was necessary to add an extra enrichment step to the procedure. In that case, 2 ml of filtered sewage in 10 ml broth were inoculated with 1 ml of a pure culture of bacteria. The culture the incubated overnight at 28°C. The enriched culture was centrifuged at 4,500 rpm for 15 minutes and the supernatant was filtered through a 0.2 um filter and spread on seeded plates as before. These were removed from the agar and inoculated into broth cultures of the appropriate bacterium to produce large quantities of phage specific for this micro-organism.

### 2. Growth and Purification

Individual plaques were removed from the agar plates and inoculated into broths seeded with E. coli, Proteus mirabilis, Vibrio flurvalis, Citrobacter freundii, Enterobacter agglomerans, E. vulneris or Pseudomonas fluorescens. The broths were incubated at 37°C until complete lysis occurred. The phage titre was estimated using the standard seeded plate method. If the titre was less than 10⁸/ml, the phage was used again to re-inoculate more seeded broths and the procedure was repeated until a titre of more than 10¹⁰/ml was obtained.

The cultures were treated with chloroform at a concentration of 10 ml per 500 ml broth, to release any remaining phage. Then the phages were purified using the method of Sambrook (Sambrook, J., Fritsch, E.F. and Maniatis, T. Molecular Cloning, a laboratory manual, 2nd Edition, Cold Spring Harbor Laboratory Press). After the cesium chloride density gradient centrifugation, the band containing the bacteriophage was stored at 4°C.

The purity of the bacteriophage was checked by extracting the DNA (Sambrook, p. 2.80) and demonstrating single bands on agarose gel electrophoresis (Sambrook, p. 6.8 to 6.13). The purity was also checked by electron microscopy. After removing the cesium chloride by dialysis, a small sample of phage preparation was dropped onto a carbon coated hydrophilic grid. After air drying, the phage particles were either positively stained with 2% (w/v) uranyl acetate or negatively stained with 2% (w/v) methylamine tungstate. After drying, the grids were observed using a transmission electron microscope.

Following the above procedure, bacteriophages against E. coli, Proteus mirabilis, Vibrio flurvalis, Citrobacter freundii, Enterobacter agglomerans, E. vulneris and Pseudomonas fluorescens were isolated and grown in bulk.

The bacteriophages against E. coli and E. vulneris were similar in morphology to the T-even phages. However, many other morphologies were observed, e.g. the phage against Enterobacter agglomerans had a round head and a very large collar.

An aqueous composition was prepared containing 10³ particles of a bacteriophage against E. coli, 1% Tween 80 and 0.01% sodium chloride. The composition was stored for two months at room temperature and it was found that the infectivity was essentially unchanged.

The above composition was brought into contact with a ceramic tile, coated with a layer of E. coli cells. After treatment with the composition containing phage the E. coli on the tile was significantly reduced, the composition without phage did not have this effect.

### EXAMPLES 8-10: Oral Bacteriophages

Fresh, stimulated human saliva was centrifuged at 10,000 rpm in a Sorvall RC-5 centrifuge for 5 minutes to remove debris. The supernatant was collected and 10 ml of chloroform was added per 500 ml supernatant. The mixture was incubated for one hour at 37°C with gentle shaking, to lyse any remaining bacteria. 0.1 ml of the treated saliva was mixed with 2 ml of molten BHI agar (45°C) and 0.1 ml of a culture of an oral organisms identified as Streptococcus mutans, Actinomyces viscosus and Streptococcus sanguis, respectively, taken from a human volunteer. The mixtures were overlayed onto a blood agar plate and incubated for 3 days at 37°C under suitable conditions for the growth of the organism, e.g. 20% CO₂ for S. mutans. After incubation, several plaques could be seen on the agar overlay.

Following the above procedure, bacteriophages against S. mutans, A. viscosus and S. sanguis were isolated and grown in bulk.

### EXAMPLE 11: Skin Bacteriophages

Following a procedure analogous to the procedure of the above examples, a bacteriophage was isolated from human skin against the micro-organism Staphylocosccus epidermis. It was found by electron-microscopy that it had a polyhedral head, no collar and a very long flexible tail

## Claims

1. Aqueous, antibacterial composition to prevent or control the growth of hygienically undesirable bacteria, comprising a nonionic surfactant, a neutral salt and at least 100 particles/ml of a bacteriophage capable of lysing one or more type of said undesirable bacteria.

2. Composition according to claim 1, comprising 0.01-15%wt surfactant.

3. Composition according to claim 2, comprising 0.1-3%wt surfactant.

4. Composition according to claim 1 wherein the surfactant is selected from the group comprising Tween 80, 20, 81 and Dobanol 23-6.5.

5. Composition according to claim 1 comprising a neutral salt at a level of 0.01-2%wt.

6. Composition according to claim 5 wherein the neutral salt is sodium chloride at a level of 0.1-1.0%.

7. Composition according to claim 1 wherein the bacteriophages which are capable of acting against bacteria, selected from the group comprising faecal bacteria, oral bacteria and/or skin bacteria.

8. Composition according to claim 7 wherein the bacteria are selected from the group comprising E. coli, Proteus mirablis, Vibrio flurvalis, Citrobacter freundii, Enterobacter aggomerans, E. vulneris, Psedomonas fluorescens, Streptococcus mutans, Actinomyces viscosus, Streptococcus sanguis, Bacterioides gingivalis, Haemophilus actinomycetemcomitans, Staphylococcus epidermis, Propionibacterium acnes and P. ovale

9. Composition according to claim 1 comprising at least 1000 particles/ml of bacteriophage capable of infecting bacteria.

10. Composition according to claim 1 comprising a thickening agent.

11. Composition according to claim 1 comprising a binder and an abrasive.

## Patentansprüche

1. Wäßrige, antibakterielle Zusammensetzung zur Verhinderung oder Kontrolle des Wachstums hygienisch unerwünschter Bakterien, welche ein nichtionisches Tensid, ein neutrales Salz und mindestens 100 Teilchen/ml eines zur Lyse eines oder mehrerer Typen besagter Bakterien fähigen Bacteriophagen umfaßt.

2. Zusammensetzung gemäß Anspruch 1, welche 0,01 - 15 Gew.-% Tensid umfaßt.

3. Zusammensetzung gemäß Anspruch 2, welche 0,1 - 3 Gew.-% Tensid umfaßt.

4. Zusammensetzung gemäß Anspruch 1, worin das Tensid aus der Gruppe ausgewählt wird, die Tween 80, 20, 81 und Dobanol 23-6.5 umfaßt.

5. Zusammensetzung gemäß Anspruch 1, welche ein neutrales Salz in einer Höhe von 0,01 - 2 Gew.-% umfaßt.

6. Zusammensetzung gemäß Anspruch 5, worin das neutrale Salz Natriumchlorid in einer Höhe von 0,1 - 1,0 Gew.-% ist.

7. Zusammensetzung gemäß Anspruch 1, worin der Bacteriophage, der zur Wirkung gegen Bakterien fähig ist, aus der Gruppe ausgewählt wird, die fäkale Bakterien, orale Bakterien und/oder Hautbakterien umfaßt.

8. Zusammensetzung gemäß Anspruch 7, worin die Bakterien aus der Gruppe ausgewählt werden, die E. coli, Proteus mirabilis, Vibrio flurvalis, Citrobacter freundii, Enterobacter agglomerans, E. vulneris, Pseudomonas fluorescens, Streptococcus mutans, Actinomyces viscosus, Streptococcus sanguis, Bacterioides gingivalis, Haemophilus actinomycetemcomitans, Staphylococcus epidermis, Propionibacterium acnes und P. ovale umfaßt.

9. Zusammensetzung gemäß Anspruch 1, welche mindestens 1000 Teilchen/ml eines zur Infizierung von Bakterien fähigen Bacteriophagen umfaßt.

10. Zusammensetzung gemäß Anspruch 1, welche ein Verdickungsmittel umfaßt.

11. Zusammensetzung gemäß Anspruch 1, welche ein Bindemittel und ein Schleifmittel umfaßt.

## Revendications

1. Composition antibactérienne aqueuse pour prévenir ou maîtriser la croissance de bactéries hygiéniquement indésirables, comprenant un agent tensioactif non-ionique, un sel neutre et au moins 100 particules/ml d'un bactériophage capable de lyser un ou plusieurs types desdites bactéries indésirables.

2. Composition selon la revendication 1, comprenant 0,01-15% en poids d'agent tensio-actif.

3. Composition selon la revendication 2, comprenant 0,1-3% en poids d'agent tensio-actif.

4. Composition selon la revendication 1 dans laquelle l'agent tensio-actif est choisi dans le groupe comprenant les Tween 80, 20, 81 et le Dobanol 23-6.5.

5. Composition selon la revendication 1 comprenant un sel neutre à un niveau de 0,01-2% en poids.

6. Composition selon la revendication 5 dans laquelle le sel neutre est le chlorure de sodium à un niveau de 0,1-1,0%.

7. Composition selon la revendication 1 dans laquelle les bactériophages sont capables d'agir contre des bactéries choisies dans le groupe comprenant les bactéries fécales, les bactéries orales et/ou les bactéries de la peau.

8. Composition selon la revendication 7 dans laquelle les bactéries sont choisies dans le groupe comprenant E.coli, Proteus mirabilis, Vibrio flurvalis, Citrobacter freundii, Enterobacter aggomerans, E. vulneris, Pseudomonas fluorescens, Streptococcus mutans, Actinomyces viscosus, Streptococcus sanguis, Bacteroides gingivalis, Haemophilus actinomycetemcomitans, Staphylococcus epidermis, Propionibacterium acnes et P. ovale.

9. Composition selon la revendication 1 comprenant au moins 1000 particules/ml de bactériophage capable d'infecter les bactéries.

10. Composition selon la revendication 1 comprenant un agent épaississant.

11. Composition selon la revendication 1 comprenant un liant et un abrasif.
